# EUROPEAN PATENT APPLICATION

(11) **EP 4 174 083 A1**
(43) Date of publication of application: **03.05.2023**
(21) Application number: 21205626.1
(22) Date of filing: 29.10.2021
(51) Int. Cl.: C07K 16/10, A61K 39/00, A61P 31/14

(54) **NEUTRALIZING MONOCLONAL ANTIBODIES AGAINST SARBECOVIRUSES**

(71) Applicant: Ospedale San Raffaele S.r.l., 20132 Milano (IT)
(72) Inventor: MANCINI, Nicasio, 20063 Cernusco sul Naviglio (MI) (IT); CLEMENTI, Nicola, 60019 Senigallia (AN) (IT); CLEMENTI, Massimo, 20129 Milano (IT); CRISCUOLO, Elena, 20054 Segrate (MI) (IT); SISTI, Sofia, 03039 Sora (FR) (IT); LIBERA, Martina, 22072 Cermenate (CO) (IT)
(74) Representative: Calogero, Ida

(57) **Abstract**

The present invention provides neutralizing monoclonal antibodies targeting the spike protein of Sarbecoviruses, such as Severe Acute Respiratory Syndrome-Coronavirus-2 (SARS-CoV-2 or COVID-19). The monoclonal antibodies of the invention can inhibit or neutralize SARS-CoV-2 activity and advantageously be used for treating, preventing or diagnosing COVID-19 infection in humans due to their significant cross-reactivity among SARS-CoV-2 variants.

## Description

### BACKGROUND

Coronaviridae family comprises a large number of virus species that are further classified in four genera. Seven species are of human interest and are highly diversified in terms of adaptation, pathogenicity and diffusion. Indeed, four species are endemic and highly adapted to humans (HCoV-229E, HCoV-NL63, HCoV-OC43 and HCoV-HKU1), two are epidemic (MERS-CoV and the extinct SARS-CoV) and one pandemic, the recently emerged and currently circulating SARS-CoV and SARS-CoV-2 belong to the same genus (Betacoronavirus) and subgenus (Sarbecovirus) but are phylogenetically distinct and have strikingly different features in terms of infectivity and clinical signs. These aspects demonstrate the difficulties in thoroughly sampling the entire Coronaviridae family necessary to implement an effective surveillance program and therefore stress the need to have therapeutic strategies with broad activity on CoVs.

Commonly, the ideal target to inhibit a virus replication is represented by the proteins that mediate entry because this prevents cell infection. Entry inhibitors can thus be used as effective therapeutics and, whether endowed with long half-life, in prophylaxis. The relevance of inhibiting virus entry is demonstrated by the fact that most neutralizing antibodies elicited by natural infection or vaccination recognizes fundamental regions of the viral proteins involved in entry.

Also, the long half-life of monoclonal antibodies (mAbs) allows their use in prophylaxis. While immunization will be the most effective approach to limit and possibly eradicate SARS-CoV-2, the identification of neutralizing mAbs (nAbs) will be fundamental to address vaccines intrinsic limitations regarding the delayed response, the fraction of non-responders and the impossibility to vaccinate immunocompromised individuals. Also, there are several open questions regarding a long-lasting humoral immunity against SARS-CoV-2 and the effect of variants continuously emerging that may partially hamper vaccination efficacy in the long run. Altogether, neutralizing mAbs against SARS-CoV-2 are and will be a fundamental therapeutic and prophylactic tool and the availability of a panel of diverse mAbs will help coping with the emergence of new variants. Furthermore, since a few mAbs naturally cross-neutralize SARS-CoV and SARS-CoV-2 and recently an in vitro matured mAb (named ADG-2) neutralizing several Sarbecoviruses has been described, there is the possibility to identify cross-reactive mAbs.

The entire entry process of all CoVs into host cells is mediated by the spike (S), a large transmembrane glycoprotein protruding from the virus envelope. The spike is a homotrimer in which each monomer is produced as a single protein (S0) that is cleaved by host proteases into the S1 and S2 subunits. S1 is located apically and contains the receptor binding domain (RBD), therefore it mediates the attachment phase by interacting with host proteins. S2 forms the spike stalk that contains the fusion peptide, the transmembrane domain, and the heptad repeats (HR1 and HR2), all necessary to fuse the virus envelope to the host membranes.

CoVs entry is a multistep process that involves receptor engagement of all three RBDs in each spike trimer, the proteolytic cleavage of at least one specific spike site and the subsequent fusion.

SARS-CoV-2, in analogy with SARS-CoV and several animal CoVs, specifically binds to the angiotensin-converting enzyme 2 (ACE2). The binding event is necessary but not sufficient to mediate entry, as SARS-CoV-2 S requires to be processed by host proteases that also specifically determine the virus entry site. Indeed, if the spike is cleaved by furin and TMPRSS2, SARS-CoV-2 fusion happens at the plasma membrane, conversely it enters from the endosomal compartment, where the spike is cleaved by cathepsins. Once the spike is completely engaged and cleaved, S1 dissociates from S2 allowing the exposure of the fusion peptide and its insertion in the host membranes. The final molecular event leading to fusion is a dramatic conformational change of S2 that involves the entire subunit and is driven by the refolding of HR1 and HR2. Notably, all events from binding to fusion require significant structural rearrangements and therefore can potentially be the target of neutralizing nAbs.

The spike protein of CoVs is the most prominent protein exposed on virions surface and the main target of the humoral response.

Characterization of sera from SARS-CoV-2 infected individuals has identified several neutralizing epitopes located on the spike protein that either overlap with domains fundamental for its function or indirectly inhibit the conformational changes required to mediate attachment and fusion.

Most of the neutralizing epitopes identified so far are conformational and located in the RBD; all nAbs binding to it can be grouped in four classes, characterized by different modes of action. Class 1 and class 2 antibodies directly hamper the interaction between ACE2 and the spike by recognizing the same subdomain in the RBD, the so-called receptor binding motif (RBM), while class 3 and 4 bind cryptic epitopes that indirectly block the correct RBM exposure or the downstream conformational changes required for fusion.

The broadly neutralizing mAb ADG-2, albeit with a unique binding mode, recognizes an epitope partially shared with class 1 nAbs, highlighting how even minor differences in terms of residues involved and angle of approach can dramatically impact the biologic activity of a nAb. At a lower frequency, nAbs recognizing other epitopes have been identified as well. Indeed, strong neutralization has been documented to a mAb that binds to a region (N-terminal domain -NTD) in S1 not directly involved in entry.

Also, the presence in patient sera of neutralizing antibodies recognizing linear epitopes located downstream of the RBD or overlapping the fusion peptide has been identified. See Table 1 of Lanying Du et al., 2021 [1] for reference, summarizing representative human neutralizing monoclonal antibodies (nAbs) against SARS-CoV-2 and the relative target antigens (i.e. RBD, NTD, etc.).

Altogether, while the neutralizing response in patient sera is dominated by antibodies specific for the RBD, several other spike domains can be effectively targeted. Worth mentioning, the RBD, and S1 in general, are the spike most variable domains, both considering SARS-CoV-2 variants continuously emerging and among CoVs belonging to the same sub-genus.

Therefore, there is an urgent need of and extreme interest for nAbs targeting conserved spike protein domains, as they reasonably have a broader spectrum of neutralizable isolates/species.

Therapeutic mAbs for COVID-19 treatment have been developed in accelerated time and the pace has been unprecedented for any disease.

Currently, 8 SARS-CoV-2 RBD-specific potent nAbs have been approved by the Food and Drug Administration (FDA) under an emergency use authorization (EUA) to treat COVID-19 non-hospitalized patients at high risk of severe illness.

The following COVID-19 mAbs are in clinical use: bamlanivimab (LY-CoV555) [2]; a combination of bamlanivimab (LY-CoV555) and etesevimab (LY-CoV016 or JS016) [3] from Eli Lilly; a combination of casirivimab (REGN10933) and Imdevimab (REGN10987) [4] from Regeneron (REGEN-COV); a combination cilgavimab (COV2-2130 or AZD1061) and tixagevimab (COV2-2196 or AZD8955) [5] from AstraZeneca; monotherapy-based nAbs sotrovimab (VIR-7831) [6] from GSK and Vir Biotechnology; and regdanvimab (CT-P59) [7] from Celltrion. Another set of monotherapy and combination Nabs-based therapies are under Phase III trials: 2B04 [8] and 47D11 [9] from AbbVie; BRII-196 and BRII-198 from Brii Biosciences [10]; and TY027 from Tychan are also in Phase III trials [10].

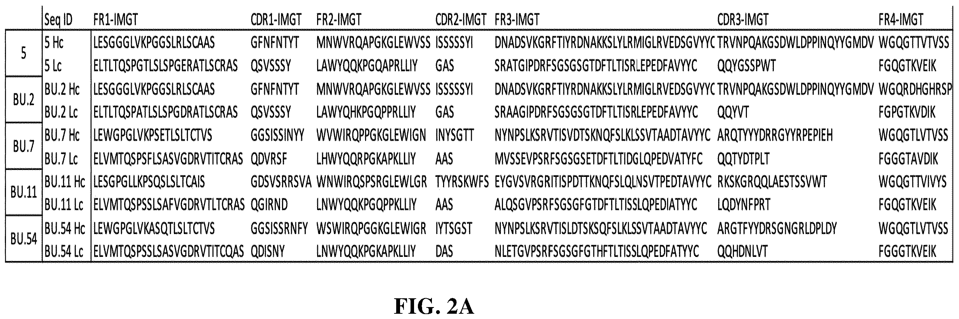

A comprehensive list of nAbs that are currently in Phase I, II, and III trials and in clinic is summarized in Figure 2A of Kumar S. et al., 2021 [11], herein included as reference.

Several SARS-CoV-2 variants are being reported from different parts of the world. According to the World Health Organization (WHO), a recognized mutation is elevated to a "variant of concern" (VOC) when the acquisition of a new mutation allows for increased viral transmission, increased fatality, and a significant decrease in the effectiveness of therapy and vaccines. A "variant of interest" (VOI) is a variant with a new mutation capable of affecting disease severity, transmissibility, immune and diagnostic escape.

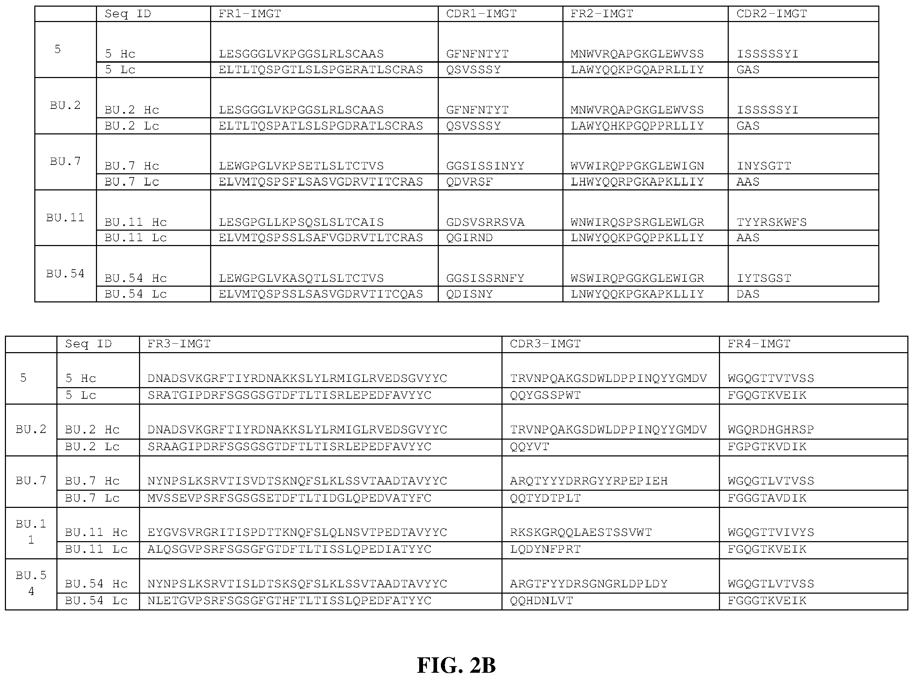

The current variants of concern are Alpha (B.1.1.7, identified in the United Kingdom) [12], Beta (B.1.351, identified in South Africa) [13], Gamma (P.1, identified in Brazil) [14], and Delta (B.1.617.2, identified in India) [15]. Current variants of interest are Eta (B.1.525, identified in UK/Nigeria), Iota (B.1.526, identified in the United States of America) [16], Kappa (B.1.617.1, identified in India) [15], and Lambda (C.37, identified in Peru) [17]. See for reference Figure 2B of Kumar S. et al., 2021 [11] providing an exhaustive list of mutations present in the current SARS-Cov-2 VOCs and VOIs.

Ideally, an effective antiviral therapeutic strategy should have the ability to prevent infection/disease by new variants while simultaneously maintaining breadth against existing multiple viral strains/variants. Recent studies have reported that many NTD-specific NAbs are relatively less effective to all emerging variants, whereas RBD-specific NAbs are variably effective against emerging variants and VOCs. The majority of the potent therapeutic nAbs as monotherapy showed complete abrogation or reduced neutralizing activity against SARS-CoV-2 emerging variants that contain the E484K/Q or L452R mutations.

For example, Bamlanivimab (LY-CoV555) was ineffective against all VOCs and thus was no longer considered for EUA. Currently, combination therapies comprising a cocktail of nAbs targeting distinct nonoverlapping epitopes on RBD have demonstrated exceptional potency and promising correlates of protection against SARS-CoV-2 and its variants. See for reference Additionally, newly identified RBD core-binding nAbs SARS2-38 and LY-CoV1404 as monotherapy potently neutralize all variants of concern of SARS-CoV-2.

The authors of the present invention have now identified neutralizing antibodies targeting conserved regions of the spike protein of SARS-CoV- 2 as promising and attractive therapeutic candidates to be used alone or in combination to tackle the disease burden caused by SARS-CoV-2 in all the VOCs and VOIs known sofar.

Therefore, it is an object of the present invention an isolated antibody or antibody fragment binding a conserved region of the spike protein of Sarbecoviruses, characterized by an antigen binding site comprising:
a) a heavy chain variable sequence comprising:
   i) a CDR1 comprising an amino acid sequence selected from the group consisting of SEQ ID NO:1, SEQ ID NO:2, SEQ ID NO:3 and SEQ ID NO:4;
   ii) a CDR2 comprising an amino acid sequence selected from the group consisting of SEQ ID NO:5, SEQ ID NO:6, SEQ ID NO:7 and SEQ ID NO:8;
   iii) a CDR3 comprising an amino acid sequence selected from the group consisting of SEQ ID NO:9, SEQ ID NO:10, SEQ ID NO:11 and SEQ ID NO:12;
   and/or
b) a light chain variable sequence comprising:
   i) a CDR1 comprising an amino acid sequence selected from the group consisting of SEQ ID NO:13, SEQ ID NO:14, SEQ ID NO:15 and SEQ ID NO:16;
   ii) a CDR2 comprising an amino acid sequence selected from the group consisting of GAS, AAS and DAS;
   iii) a CDR3 comprising an amino acid sequence selected from the group consisting of SEQ ID NO:20, SEQ ID NO:21, SEQ ID NO:22, SEQ ID NO:23 and SEQ ID NO:24.

Complementary determining regions (CDRs; CDR1-CDR3) of the variable domain of heavy and constant regions and framework regions (FRs; FR1-FR4) characterizing the sequence of the isolated antibody or antibody fragment of the invention have been determined according to IMGT nomenclature.

According to preferred embodiment the heavy chain variable sequence the antibody or antibody fragment of the invention, further comprises:
a) a framework region (FR1) comprising an amino acid sequence selected from the group consisting of SEQ ID NO:25, SEQ ID NO:26, SEQ ID NO:27 and SEQ ID NO:28;
b) a framework region (FR2) comprising an amino acid sequence selected from the group consisting of SEQ ID NO:29, SEQ ID NO:30, SEQ ID NO:31 and SEQ ID NO:32;
c) a framework region (FR3) comprising an amino acid sequence selected from the group consisting of SEQ ID NO:33, SEQ ID NO:34, SEQ ID NO:35 and SEQ ID NO:36;
d) a framework region (FR4) comprising an amino acid sequence selected from the group consisting of SEQ ID NO:37, SEQ ID NO:38, SEQ ID NO:39 and SEQ ID NO:40;
and/or
wherein the light chain variable sequence further comprises:
a) a framework region (FR1) comprising an amino acid sequence selected from the group consisting of SEQ ID NO:41, SEQ ID NO:42, SEQ ID NO:43; SEQ ID NO:44 and SEQ ID NO:45;
b) a framework region (FR2) comprising an amino acid sequence selected from the group consisting of SEQ ID NO:46, SEQ ID NO:47, SEQ ID NO:48, SEQ ID NO:49 and SEQ ID NO:50;
c) a framework region (FR3) comprising an amino acid sequence selected from the group consisting of SEQ ID NO:51, SEQ ID NO:52, SEQ ID NO:53, SEQ ID NO:54 and SEQ ID NO:55;
d) a framework region (FR4) comprising an amino acid sequence selected from the group consisting of SEQ ID NO:56, SEQ ID NO:57, SEQ ID NO:58 and SEQ ID NO:59.

According to preferred embodiments of the present invention the antibody or antibody fragment is characterized by an antigen binding site comprising:
a) a heavy chain variable sequence comprising a CDR1 comprising the amino acid sequence SEQ ID NO:1; a CDR2 comprising the amino acid sequence SEQ ID NO:5; a CDR3 comprising the amino acid sequence SEQ ID NO:9 and/or a light chain variable sequence comprising a CDR1 comprising the amino acid sequence SEQ ID NO:13; a CDR2 comprising the amino acid sequence GAS; a CDR3 comprising the amino acid sequence SEQ ID NO:20. According to another preferred embodiment said heavy chain variable sequence may further comprises: a framework region FR1 comprising SEQ ID NO:25; a framework region FR2 comprising SEQ ID NO:29; a framework region FR3 comprising SEQ ID NO:33 and a framework region FR4 comprising SEQ ID NO:37; said light chain variable sequence may further comprises a framework region FR1 comprising SEQ ID NO:41, a framework region FR2 comprising SEQ ID NO:46, a framework region FR3 comprising SEQ ID NO:51, and a framework region FR4 comprising SEQ ID NO:56;
b) a heavy chain variable sequence comprising: a CDR1 comprising the amino acid sequence SEQ ID NO:1; a CDR2 comprising the amino acid sequence SEQ ID NO:5; a CDR3 comprising the amino acid sequence SEQ ID NO:9 and/or a light chain variable sequence comprising a CDR1 comprising the amino acid sequence SEQ ID NO:13; a CDR2 comprising the amino acid sequence GAS; a CDR3 comprising the amino acid sequence SEQ ID NO:21. According to another preferred embodiment said heavy chain variable sequence may further comprises a framework region FR1 comprising SEQ ID NO:25; a framework region FR2 comprising SEQ ID NO:29; a framework region FR3 comprising SEQ ID NO:33 and a framework region FR4 comprising SEQ ID NO:38; said light chain variable sequence may further comprises a framework region FR1 comprising SEQ ID NO:42; a framework region FR2 comprising SEQ ID NO:47; a framework region FR3 comprising SEQ ID NO:52, and a framework region FR4 comprising SEQ ID NO:57;
c) a heavy chain variable sequence comprising a CDR1 comprising the amino acid sequence SEQ ID NO:2; a CDR2 comprising the amino acid sequence SEQ ID NO:6; a CDR3 comprising the amino acid sequence SEQ ID NO:10 and/or a light chain variable sequence comprising a CDR1 comprising the amino acid sequence SEQ ID NO:14; a CDR2 comprising the amino acid sequence AAS; a CDR3 comprising the amino acid sequence SEQ ID NO:22. According to another preferred embodiment said heavy chain variable sequence may further comprises a framework region FR1 consisting of SEQ ID NO:26; a framework region FR2 consisting of SEQ ID NO:30 a framework region FR3 comprising SEQ ID NO:34, and a framework region FR4 comprising SEQ ID NO:39; said light chain variable sequence may further comprises a framework region FR1 comprising SEQ ID NO:43; a framework region FR2 comprising SEQ ID NO:48; a framework region FR3 comprising SEQ ID NO:53 and a framework region FR4 comprising SEQ ID NO:58;
d) a heavy chain variable sequence comprising a CDR1 comprising the amino acid sequence SEQ ID NO:3; a CDR2 comprising the amino acid sequence SEQ ID NO:7; a CDR3 comprising the amino acid sequence SEQ ID NO:11 and/or a light chain variable sequence comprising a CDR1 comprising the amino acid sequence SEQ ID NO:15; a CDR2 comprising the amino acid sequence AAS; a CDR3 comprising the amino acid sequence SEQ ID NO:23. According to another preferred embodiment said heavy chain variable sequence may further comprises a framework region FR1 comprising SEQ ID NO:27; a framework region FR2 comprising SEQ ID NO:31 a framework region FR3 comprising SEQ ID NO:35 and a framework region FR4 consisting of SEQ ID NO:40; said light chain variable sequence may further comprises a framework region FR1 comprising SEQ ID NO:44; a framework region FR2 comprising SEQ ID NO:49; a framework region FR3 comprising SEQ ID NO:54 and a framework region FR4 comprising SEQ ID NO:56;
e) a heavy chain variable sequence comprising a CDR1 comprising the amino acid sequence SEQ ID NO:4; a CDR2 comprising the amino acid sequence SEQ ID NO:8; a CDR3 comprising the amino acid sequence SEQ ID NO:12; and/or a light chain variable sequence comprising: a CDR1 comprising the amino acid sequence SEQ ID NO:16; a CDR2 comprising the amino acid sequence DAS; a CDR3 comprising the amino acid sequence SEQ ID NO:24. According to another preferred embodiment said heavy chain variable sequence may further comprises a framework region FR1 comprising SEQ ID NO:28; a framework region FR2 comprising SEQ ID NO:32; a framework region FR3 comprising SEQ ID NO:36 and a framework region FR4 comprising SEQ ID NO:39; said light chain variable sequence may further comprises a framework region FR1 comprising SEQ ID NO:45; a framework region FR2 comprising SEQ ID NO:50; a framework region FR3 comprising SEQ ID NO:55; and a framework region FR4 comprising SEQ ID NO:59.

According to a preferred embodiment of the invention the antibody or antibody fragment (clone Fab 5#) of the invention comprises an heavy chain variable sequence comprising the amino acid sequence: 
LESGGGLVKPGGSLRLSCAASGFNFNTYTMNWVRQAPGKGLEWVSSISSSS
SYIDNADSVKGRFTIYRDNAKKSLYLRMIGLRVEDSGVYYCTRVNPQAKGS
DWLDPPINQYYGMDVWGQGTTVTVSS (SEQ ID NO:60)
 and/or a light chain variable sequence comprising the amino acid sequence:

ELTLTQSPGTLSLSPGERATLSCRASQSVSSSYLAWYQQKPGQAPRLLIYGA
SSRATGIPDRFSGSGSGTDFTLTISRLEPEDFAVYYCQQYGSSPWTFGQGTK
VEIK (SEQ ID NO: 61).

In another preferred embodiment the antibody or antibody fragment (clone Fab BU.2) of the invention comprises an heavy chain variable sequence comprising the amino acid sequence: 
LESGGGLVKPGGSLRLSCAASGFNFNTYTMNWVRQAPGKGLEWVSSISSSS
SYIDNADSVKGRFTIYRDNAKKSLYLRMIGLRVEDSGVYYCTRVNPQAKGS
DWLDPPINQYYGMDVWGQRDHGHRSP (SEQ ID NO: 62)
 and a light chain variable sequence comprising the amino acid sequence:

ELTLTQSPATLSLSPGDRATLSCRASQSVSSSYLAWYQHKPGQPPRLLIYGA
SSRAAGIPDRFSGSGSGTDFTLTISRLEPEDFAVYYCQQYVTFGPGTKVDIK
(SEQ ID NO: 63)

According to another preferred alternative embodiment of the invention the isolated antibody or antibody fragment (clone Fab BU.7) of the invention comprises an heavy chain variable sequence comprising the amino acid sequence: 
LEWGPGLVKPSETLSLTCTVSGGSISSINYYWVWIRQPPGKGLEWIGNINYS
GTTNYNPSLKSRVTISVDTSKNQFSLKLSSVTAADTAVYYCARQTYYYDRR
GYYRPEPIEHWGQGTLVTVSS (SEQ ID NO:64)
 and/or a light chain variable sequence comprising the amino acid sequence:

ELVMTQSPSFLSASVGDRVTITCRASQDVRSFLHWYQQRPGKAPKLLIYAA
SMVSSEVPSRFSGSGSETDFTLTIDGLQPEDVATYFCQQTYDTPLTFGGGTA
VDIK (SEQ ID NO:65).

In another embodiment of the invention the isolated antibody or antibody fragment of the invention (clone Fab BU.11) comprises an heavy chain variable sequence comprising the amino acid sequence: 
LESGPGLLKPSQSLSLTCAISGDSVSRRSVAWNWIRQSPSRGLEWLGRTYYR
SKWFSEYGVSVRGRITISPDTTKNQFSLQLNSVTPEDTAVYYCRKSKGRQQ
LAESTSSVWTWGQGTTVIVYS (SEQ ID NO:66)
 and/or a light chain variable sequence comprising the amino acid sequence:

ELVMTQSPSSLSAFVGDRVTLTCRASQGIRNDLNWYQQKPGQPPKLLIYAA
SALQSGVPSRFSGSGFGTDFTLTISSLQPEDIATYYCLQDYNFPRTFGQGTKV
EIK (SEQ ID NO:67).

Still in another alternative preferred embodiment of the invention the isolated antibody or antibody fragment of the invention (clone Fab BU.54) comprises an heavy chain variable sequence comprising the amino acid sequence: 
LEWGPGLVKASQTLSLTCTVSGGSISSRNFYWSWIRQPGGKGLEWIGRIYTS
GSTNYNPSLKSRVTISLDTSKSQFSLKLSSVTAADTAVYYCARGTFYYDRSG
NGRLDPLDYWGQGTLVTVS (SEQ ID NO:68)
 and/or a light chain variable sequence comprising the amino acid sequence:

ELVMTQSPSSLSASVGDRVTITCQASQDISNYLNWYQQKPGKAPKLLIYDA
SNLETGVPSRFSGSGFGTHFTLTISSLQPEDFATYYCQQHDNLVTFGGGTKV
EIK (SEQ ID NO:69).

It is a further object of present invention a nucleotide sequence encoding for the heavy chain variable region and/or the light chain variable region of the antibody or antibody fragment of the invention above listed.

In a preferred embodiment the nucleotide sequences of the invention are selected from the group consisting of:
Heavy chain (clone Fab#5): 
ctcgagtctgggggaggcctggtcaagcctggggggtccctgagactctcctgtgcagcctctggattcaacttcaatac
ctataccatgaactgggtccgccaggctccagggaaggggctggagtgggtctcatccattagtagtagtagtagttaca
tagacaacgcagactcagtgaagggccgattcaccatctacagagacaacgccaagaagtcactgtatctgcgaatgat
cggcctgagagtcgaggactccggtgtgtattactgtacgcgagtgaacccccaggccaaagggtcggactggttgga
tccccccatcaaccaatactacggtatggacgtctggggccaagggaccacggtcaccgtctcctca (SEQ ID
NO:70)
Light chain (Fab#5): 
gagctcacactcacgcagtctccaggcaccctgtctttgtctccaggggaaagagccaccctctcctgcagggccagtc
agagtgttagcagcagctatttagcctggtaccagcagaaacctggccaggctcccaggctcctcatctatggtgcatcc
agcagggccactggcatcccagacaggttcagtggcagtgggtctgggacagacttcactctcaccatcagcagactg
gagcctgaagattttgcagtgtattactgtcagcagtatggtagctcaccgtggacgttcggccaagggaccaaggttga
aatcaaa (SEQ ID NO:71)
Heavy chain (Fab BU.2) 
ctcgagtctgggggaggcctggtcaagcctggggggtccctgagactctcctgtgcagcctctggattcaacttcaatac
ctataccatgaactgggtccgccaggctccagggaaggggctggagtgggtctcatccattagtagtagtagtagttaca
tagacaacgcagactcagtgaagggccgattcaccatctacagagacaacgccaagaagtcactgtatctgcgaatgat
cggcctgagagtcgaggactccggtgtgtattactgtacgcgagtgaacccccaggccaaagggtcggactggttgga
tccccccatcaaccaatactacggtatggacgtctggggccaaagggaccacggtcaccggtctcct
(SEQ ID NO:72)
Light chain (Fab BU.2) 
gagctcacactcacgcagtctccagccaccctgtctttgtctccaggggatagagccaccctctcctgcagggccagtc
agagtgttagcagcagttacttagcctggtaccagcacaaacctggccagccccccaggctcctcatctatggtgcatcc
agcagggccgccggcatcccagacaggttcagtggcagtgggtctgggacagacttcactctcaccatcagcagact
ggagcctgaagattttgcagtgtattactgtcagcagtacgtcactttcggccctgggaccaaagtggatatcaaa
(SEQ ID NO:73)
Heavy chain (Fab BU.7) 
Ctcgagtggggcccaggactggtgaagccttcggagaccctgtccctcacctgcactgtctctggtggctccatcagca
gcataaattactactgggtctggattcgccagcccccagggaaggggctggagtggattgggaatatcaattacagtgg
gaccaccaactacaacccgtccctcaagagtcgagtcaccatatccgtggacacgtccaagaaccagttctccctgaag
ctgagctctgtgaccgccgcagacacggctgtctattactgtgcgagacaaacgtattactatgataggcgtggttattac
cgcccggagcccattgagcactggggccagggaaccctggtcaccgtctcctca (SEQ ID NO:74)
Light chain (Fab BU.7): 
Gagctcgtgatgacacagtctccatccttcctgtctgcatctgtaggagacagagtcaccatcacttgccgggcaagtca
agatgttagaagttttttacattggtatcaacaaagaccagggaaagcccctaagttgttgatctatgctgcatccatggtgt
cgagtgaggtcccgtcaaggttcagtggcagtggatctgagacagatttcactctcaccatcgacggtctgcaacctgaa
gatgttgcaacttacttctgtcaacagacttacgacacgccgctcaccttcggcggcgggaccgcggttgacatcaaa
(SEQ ID NO:75)
Heavy chain (Fab BU.11) 
Ctcgagtcaggtccaggactgctgaagccctcgcagagcctctcactcacctgtgccatctccggagacagtgtctcta
ggagaagtgttgcttggaactggatcagacagtccccatcgagaggccttgagtggctgggaaggacatactacaggt
ccaagtggttttctgagtatggcgtatctgtgagaggtcgaataaccatcagtccagacacaaccaagaaccagttctcc
ctgcagctgaactccgtgactcccgaggacacggctgtctattactgtcgaaagtcgaaagggaggcagcagttggca
gagtctacgagttcggtatggacgtggggccaagggaccacggtcatcgtctactca (SEQ ID NO:76)
Light chain (Fab BU.11) 
gagctcgtgatgacacagtctccatcctccctgtctgcatttgtgggagatagagtcaccctcacttgccgggcaagtca
gggcattagaaatgatttaaactggtatcagcagaaaccagggcaaccccctaaactcctgatctatgctgcatccgcttt
acaaagtggcgtcccatcaaggttcagcggcagtgggtttggcacagatttcactctcaccatcagcagcctgcagcct
gaagatattgcaacttattactgtctacaagattacaatttccctcggacgttcggccaagggaccaaggtggaaatcaaa
(SEQ ID NO:77)
Heavy chain (Fab BU.54) 
ctcgagtggggcccaggactggtgaaggcttcacagaccctgtccctcacctgcactgtctctggtggctccatcagca
gtaggaatttctattggagttggatccggcagcccggcgggaagggactggagtggattgggcgtatctatacaagtgg
gagcaccaattacaatccctccctcaagagtcgagtcactatatcattagacacgtccaagagtcagttctccctgaagct
gagctctgtgaccgccgcagacacggccgtgtattattgtgcgagagggacgttttattatgataggagtggtaatggtc
gattagatccgcttgactactggggccagggaaccctggtcaccgtctcctca (SEQ ID NO:78)
Light chain (Fab BU.54)

gagctcgtgatgacacagtctccatcctccctgtctgcatctgtaggagacagagtcaccatcacttgccaggcgagtca
ggacattagcaactatttaaattggtatcagcagaaaccagggaaagcccctaagctcctgatctacgatgcatccaattt
ggaaacaggggtcccatcaaggttcagtggaagtggatttgggacacattttacgttaaccatcagcagcctgcagcct
gaagattttgcaacatattactgtcaacagcatgataatctcgtcactttcggcggagggaccaaggtggagatcaaa
(SEQ ID NO:79)

Additionally, the invention reates to an expression vector comprising at least one of the nucleotide sequence encoding for the heavy chain and/or the light chain variable region of the antibody or antibody fragment of the invention.

A further object of the invention contemplates an host cell comprising the vector above outlined.

It is another object of the invention an hybridoma comprising at least one of the nucleotide sequence encoding for the heavy chain and/or the light chain variable region of the antibody or antibody fragment of the invention.

Additionally, the invention contemplates a formulation for molecular/vector-based passive immunoprophylaxis comprising at least one of the nucleotide sequence or expression vector of the invention together with one or more pharmaceutically acceptable excipients and/or adjuvants. The vaccine formulation may be advantageously be used in the prevention of Sarbecoviruses-mediated diseases in a subject, in particular of the Severe Acute Respiratory Syndrome mediated by SARS-CoV-2.

Preferably the antibody or antibody fragment of the invention is a monoclonal antibody. In another preferred embodiment the antibody fragment is a Fab fragment. Even more preferably the antibody or antibody fragment of the invention is a human antibody.

Preferably, the antibody or antibody fragment is a human IgG or an IgG fragment selected from the group consisting of IgG1, IgG2, IgG3, IgG4 or a recombinant IgG comprising a mutated Fc portion to improve their binding and neutralization activity or all the Fc-mediated immune functions.

In alternative embodiments the antibody or antibody fragment of the invention are in a format selected among the group comprising single chain antibodies (scFv), nanobodies, bispecific antibodies, monoclonal antibodies conjugated with drugs and/or molecules influencing their pharmacokinetics and/or their delivery. According to a further embodiment the antibody or antibody fragment of the invention is labelled with a marker or conjugated with a drug.

As an example, antitumour activity has been accomplished by conjugating antibodies with different effector molecules that accomplish cell death after antibody binding and internalisation. Such effector molecules include cytotoxic agents [18, 19], bacterial or plant protein toxins (immunotoxins) [20, 21], and radiopharmaceutical agents [22].

A further object of the present invention is the use of the antibody or antibody fragment of the invention to detect the presence of a Sarbecovirus, preferably of SARS-CoV-2 in a biological sample.

The present invention further contemplates the antibody or antibody fragment of the invention for use in medical field for the treatment and/or prophylaxis of Sarbecoviruses-mediated diseases in a subject, in particular of the Severe Acute Respiratory Syndrome mediated by SARS-CoV-2.

The term "SARS-Cov-2" encompasses anyone of the variants of concern (VOCs) Alpha (B.1.1.7), Beta (B.1.351) Gamma (P.1), Delta (B.1.617.2) and D614 G (Università di Pavia) or of the variants of interest (VOIs) Eta (B.1.525), Iota (B.1.526), Kappa (B.1.617.1) and Lambda (C.37), known sofar.

The antibody or antibody fragment of the present invention may be used alone or in combination with another antibody or antibody fragment directed against different conserved regions of the spike protein. The combined use of the antibody or antibody fragment of the invention enhances the cross reactivity of the therapeutic treatment towards the different VOCs of SARS-CoV-2. The second antibody may be also a different antibody with respect to the claimed ones. Combinations of antibodies targeting different epitopes can translate into enhanced therapeutic efficacy, i.e., overcoming drug-related side effects of already approved molecules or clearing drug resistance of possible novel circulating variants.

According to a preferred embodiment the subject is an immunocompromised patient or a patient with an history of cardiovascular and/or respiratory diseases. Alternatively the patient may be an elderly patient (that is older than 70 years) or a vaccine-hesitant subject.

The antibody or antibody fragment according to the invention may be advantageously therapeutically administered in a human being also in a combination antibody cocktail.

Preferably, the antibody or antibody fragment of the invention is administered after 5- 10 days from occurrence of SARS-CoV-2 infection.

It is an object of the present invention a pharmaceutical composition comprising at least one of the antibody or antibody fragment of the invention as the active ingredient, together with one or more pharmaceutically acceptable excipients and/or adjuvants. In a preferred embodiment the antibody or antibody fragment is adapted to systemic administration by intravenous route. The invention alternatively contemplates intramuscular or subcutaneous administration route.

The present invention further relates to a pharmaceutical composition comprising at least one of the antibody or antibody fragment of the invention for use in the treatment or prophylaxis of Sarbecoviruses-mediated diseases in a subject, in particular of the Severe Acute Respiratory Syndrome mediated by SARS-CoV-2.

It is a further object of the present invention a composition or kit of parts comprising a first antibody or antibody fragment that binds a targeted conserved region of the spike protein of SARS-CoV-2 of the invention and a second antibody or antibody fragment that binds a different targeted conserved region of the spike protein of SARS-CoV-2, for the simultaneous, separate or sequential administration in a subject affected by Sarbecoviruses-mediated diseases, in particular by the Severe Acute Respiratory Syndrome mediated by SARS-CoV-2. Preferably the first antibody or antibody fragment and the second antibody or antibody fragment are in separate containers.

The present invention will now be described, for non-limiting illustrative purposes, according to preferred embodiments thereof, with particular reference to the attached figures, wherein:

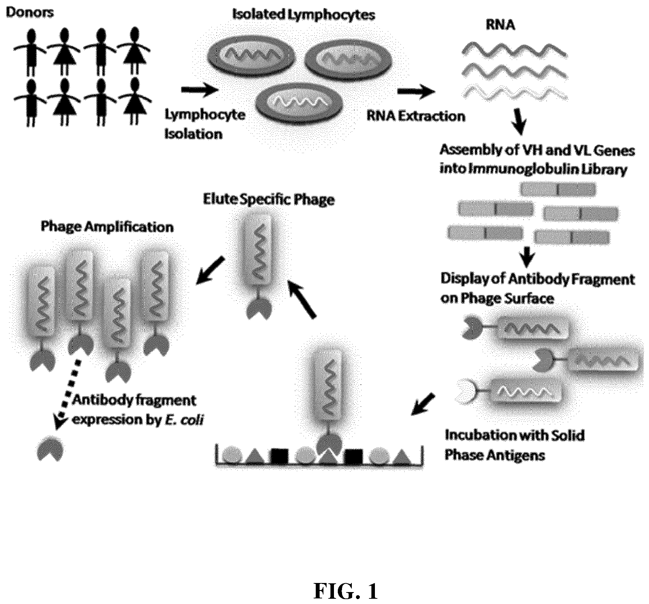

- **Figure 1** shows a general scheme for the preparation and selection of a naive recombinant antibody library. This figure indicates the general steps involved in the construction and selection of a recombinant antibody from a phage display library. The B-lymphocytes are first isolated from the blood of the donors. The total mRNA is extracted from the isolated lymphocyte cells and reverse-transcribed to complementary DNA (cDNA). The variable heavy (VH) and variable light (VL) chain antibody genes are amplified by polymerase chain reaction with gene-specific primers and assembled before cloning into a phage display vector. The resulting phages display antibody fragments on their surfaces. During selection, filamentous phage display libraries are incubated with antigens immobilized on a solid phase. The antigen-binding phages remains attached to the antigen, whereas the unbound phages are washed away. The bound phages are then eluted and amplified by infection with bacterial cells. By changing the expression host to a non-suppressor bacterial strain, the soluble antibody fragment can be expressed and secreted to the culture medium.
- **Figure 2** shows the whole amino acid (panel A) and nucleotide sequences (panel B) of the heavy chain variable region and of the light chain variable region for each clone, specifying both the framework regions FR1-FR4 and the complementary determining regions CDR1-CDR3.

- **Figure 3** shows the binding activity of the selected clones assessed by ELISA assay. The selected Fabs were tested for their binding to the recombinant protein of the different VOCs.
- **Figure 4** shows the results of immunofluorescence analysis of the binding activity of the selected clones. The selected Fabs can detect the VeroE6 cells infected by B.1.1.7, B.1.135 and P.1 viral variants.
- **Figure 5** shows neutralizing activities of clone Fab#5, both as Fab and IgG1 format (panel A) and of clones Fab BU.2, BU.7 and BU.11 (panel B) against the tested VOCs (D614G, B.1.1.7, B.1.135, P.1).

For the purpose of better illustrating the invention, the following examples are now provided, which should be considered illustrative and non-limiting examples thereof. The neutralizing nAbs of the invention have been identified on the basis of the binding affinity to SARS-CoV-2 spike or specific domains and characterized in terms of neutralizing activity and epitope specificity as described in the following examples. These aspects are fundamental to select the most promising nAbs as those endowed with the highest potency and/or recognizing conserved spike epitopes, the main parameters determining a potential cross reactive treatment effective against all the VOIs and VOIc of SARS-Cov-2 known sofar or emerging in the future.

### EXAMPLE 1: Generation of new human antibody Phage Display Libraries and selection of antibodies able to bind with high affinity the spike protein of SARS-CoV-2 through Bio-panning procedures

### MATERIALS AND METHODS

### Phage display technique

The technique focuses on the construction of a library of peptides or antibody variants, which are then selected for their affinity to the target of interest since they are fused to a phage-coat protein (see for reference **Figure 1****).**

All surface proteins of bacteriophages can be engineered for display, but the most used are pVIII and pIII from M13 filamentous phages. Each virion contains about 2700 copies of the former protein, representing almost 87% of its mass, and they are half-exposed to the environment, thus allowing an efficient display of only short-sequence peptides due to virion architecture. On the other hand, pIII can be used for larger peptides, such as the "functional portion" of antibodies, resulting only in a slight loss of infectivity in a few cases. Each library is composed by phagemid vectors containing only the sequence of a phage-coat protein fused to the peptide of interest; therefore, a helper phage with a reduced packaging efficiency is needed to obtain a population of phages both infectious and composed by modified coating proteins. The bio-panning procedure is then performed, and phages are selected for their ability to bind the antigen of interest. Many factors must be considered: library variability, target conformation, affinity, and avidity of the molecules exposed on phages. As mentioned, phage display has been widely used to find novel therapeutics against pathogens, particularly mAbs.

This has been possible through two different bio-panning strategies: using specific molecular targets, such as membrane receptors (S protein), or using whole viruses, or infected cells. However, surface antigens often present motifs able to elicit non-neutralizing mAbs and elude host immune response, so the screening procedure of the bio-panning outcome must be done properly to identify only the few effective molecules able to selectively target the antigen of interest.

### Generation of new human antibody Phage Display Libraries

Peripheral (for Fab #5) and bone marrow (for Fabs BU.2, BU.7, BU.11, BU.54) blood samples was collected from subjects previously infected by SARS-CoV-2 and subsequently vaccinated with COVID-19 vaccine (2 doses of Comirnaty, Pfizer) and used for the collection of PBMC and B-cell precursors, respectively.

The extraction of PBMCs was carried out following the Histopaque^{®}-1077 (SIGMA-ALDRICH) protocol. At the end of the procedure 10⁶ cells were lysed using Trizol Reagent (SIGMA-ALDRICH) to allow the subsequent extraction of cellular RNA using RNeasy Mini Kit (QIAGEN). Antibody phagemidic IgG libraries were generated through B-cell-mRNA reverse transcription to cDNA using SuperScript^{™} IV Reverse Transcriptase (ThermoFisher Scientific). Heavy and light chains were amplified from the obtained cDNA by using the PFU Native Polymerase enzyme (Agilent) and the primer pairs described in the following Table.

**Table 1. Primers used for the amplification of heavy and light chains**

| Heavy chain | Primer Forward | Primer Reverse |
|---|---|---|
| 1 | VH135: AGGTGCAGCTGCTCGAGTCTGG (SEQ ID NO:17) | IgG1:GCATGTACTAGTTTTGTCACAAG ATTTGG (SEQ ID NO:18) |
| 2 | VH2: CAGATCACCTTGCTCGAGTCTGG (SEQ ID NO:19) | IgG1 |
| 3 | VH4: CAGGTGCAGCTGCTCGAGTCGGG (SEQ ID NO:80) | |
| 4 | VH4b: CAGGTGCAGCT ACTCGAGTGGGG (SEQ ID NO:81) | |
| 5 | VH4gs: CAGGTGCAGCTACTCGAGTGGGGC (SEQ ID NO:82) | |
| 6 | VH6: CAGGTACAGCTGCTCGAGTCAGG (SEQ ID NO:83) | |
| | | |
| Light chain | | |
| 1 | VK1:GAGCCGCACGAGCCCGAGCTCCAGATG ACCCAGTCTCC (SEQ ID NO:84) | CK1d:GCGCCGTCTAGAATTAACACTCT CCCCTGTTGAAGCTCTTTGTGACGGGC GAACTCAG (SEQ ID NO:85) |
| 2 | VK3:GAGCCGCACGAGCCCGAGCTCGTGATG ACACAGTCTCC (SEQ ID NO:86) | CK1d |
| 3 | VK2/4:GAGCCGCACGAGCCCGAGCTCGTGAT GACCCAGTCTCC(SEQ ID NO:87) | |
| 4 | VK5:GAGCCGCACGAGCCCGAGCTCACACTC ACGCAGTCTCC (SEQ ID NO:88) | |

Then, both heavy and light variable antibody chains were cloned into the phagemidic vector pComb3XSS (PVT10572, DBA).

Phagemidic libraries were then converted into phage libraries by transforming electrocompetent Gram-negative cells expressing sexual pilus (XL1-Blue, Agilent) and superinfecting them with a helper-phage (M13K07, NEB).

The new libraries were screened by using different Bio-panning procedures with the aim of maximizing the selection of cross-reactive antibodies (antibodies recognizing more SARS-CoV-2 clinical isolates) in their Fab format.

Recombinant spike (S) proteins of different SARS-CoV-2 variants were used: D614G (University of Pavia) for Fab #5, and B.1.1.7 (40589-V08B6, Sino Biological), P.1 (40589-V08B10, Sino Biological) or B.1.135 (40589-V08B7, Sino Biological) for Fab BU.2, BU.7, BU.11, BU.54.

Five rounds of Bio-panning of the phage-display antibody libraries were carried out on recombinant Spike proteins expressed in their full-length format. Cross-selection strategies based on the sequential use of the different antigens were performed. Bacteriophages carrying on their capsid monoclonal antibodies (expressed as Fab fragments) selected through bio-panning were used to obtain the DNA codifying the antibody expressed on their surface.

DNAs codifying for the variable antibody regions present on the phage surface was sequenced and used for protein purification using E. coli expression system (XL1-Blue, Agilent) and immune-affinity chromatography (HiTrap^{®} Protein L, Merck). IgG1 format of Fab #5 (IgG #5) was obtained from Genscript through High Throughput Antibody Production Service (GenScript).

### RESULTS

Five different clones were identified: Fab #5 and Fab BU.2, BU.7, BU.11, BU.54. The amino acidic sequences of the heavy and light chain variable regions are the following:
1. Fab #5
   Hc: 
LESGGGLVKPGGSLRLSCAASGFNFNTYTMNWVRQAPGKGLEWVSSISSSS
SYIDNADSVKGRFTIYRDNAKKSLYLRMIGLRVEDSGVYYCTRVNPQAKGS
DWLDPPINQYYGMDVWGQGTTVTVSS (SEQ ID NO:60)
   Lc: 
ELTLTQSPGTLSLSPGERATLSCRASQSVSSSYLAWYQQKPGQAPRLLIYGA
SSRATGIPDRFSGSGSGTDFTLTISRLEPEDFAVYYCQQYGSSPWTFGQGTK
VEIK (SEQ ID NO:61)
2. Fab BU.2
   Hc: 
LESGGGLVKPGGSLRLSCAASGFNFNTYTMNWVRQAPGKGLEWVSSISSSS
SYIDNADSVKGRFTIYRDNAKKSLYLRMIGLRVEDSGVYYCTRVNPQAKGS
DWLDPPINQYYGMDVWGQRDHGHRSP (SEQ ID NO:62)
   Lc: 
ELTLTQSPATLSLSPGDRATLSCRASQSVSSSYLAWYQHKPGQPPRLLIYGA
SSRAAGIPDRFSGSGSGTDFTLTISRLEPEDFAVYYCQQYVTFGPGTKVDIK
(SEQ ID NO:63)
3. Fab BU.7
   Hc: 
LEWGPGLVKPSETLSLTCTVSGGSISSINYYWVWIRQPPGKGLEWIGNINYS
GTTNYNPSLKSRVTISVDTSKNQFSLKLSSVTAADTAVYYCARQTYYYDRR
GYYRPEPIEHWGQGTLVTVSS (SEQ ID NO:64)
   Lc: 
ELVMTQSPSFLSASVGDRVTITCRASQDVRSFLHWYQQRPGKAPKLLIYAA
SMVSSEVPSRFSGSGSETDFTLTIDGLQPEDVATYFCQQTYDTPLTFGGGTA
VDIK (SEQ ID NO:65)
4. Fab BU.11
   Hc: 
LESGPGLLKPSQSLSLTCAISGDSVSRRSVAWNWIRQSPSRGLEWLGRTYYR
SKWFSEYGVSVRGRITISPDTTKNQFSLQLNSVTPEDTAVYYCRKSKGRQQ
LAESTSSVWTWGQGTTVIVYS (SEQ ID NO:66)
   Lc: 
ELVMTQSPSSLSAFVGDRVTLTCRASQGIRNDLNWYQQKPGQPPKLLIYAA
SALQSGVPSRFSGSGFGTDFTLTISSLQPEDIATYYCLQDYNFPRTFGQGTKV
EIK (SEQ ID NO:67)
5. Fab BU.54
   Hc: 
LEWGPGLVKASQTLSLTCTVSGGSISSRNFYWSWIRQPGGKGLEWIGRIYTS
GSTNYNPSLKSRVTISLDTSKSQFSLKLSSVTAADTAVYYCARGTFYYDRSG
NGRLDPLDYWGQGTLVTVSS (SEQ ID NO:68)
   Lc:

ELVMTQSPSSLSASVGDRVTITCQASQDISNYLNWYQQKPGKAPKLLIYDA
SNLETGVPSRFSGSGFGTHFTLTISSLQPEDFATYYCQQHDNLVTFGGGTKV
EIK (SEQ ID NO:69)

The nucleotide sequences encoding the same are the following:
1. Fab #5
   Hc: 
ctcgagtctgggggaggcctggtcaagcctggggggtccctgagactctcctgtgcagcctctggattcaacttcaatac
ctataccatgaactgggtccgccaggctccagggaaggggctggagtgggtctcatccattagtagtagtagtagttaca
tagacaacgcagactcagtgaagggccgattcaccatctacagagacaacgccaagaagtcactgtatctgcgaatgat
cggcctgagagtcgaggactccggtgtgtattactgtacgcgagtgaacccccaggccaaagggtcggactggttgga
tccccccatcaaccaatactacggtatggacgtctggggccaagggaccacggtcaccgtctcctca
(SEQ ID NO:70)
   Lc: 
gagctcacactcacgcagtctccaggcaccctgtctttgtctccaggggaaagagccaccctctcctgcagggccagtc
agagtgttagcagcagctatttagcctggtaccagcagaaacctggccaggctcccaggctcctcatctatggtgcatcc
agcagggccactggcatcccagacaggttcagtggcagtgggtctgggacagacttcactctcaccatcagcagactg
gagcctgaagattttgcagtgtattactgtcagcagtatggtagctcaccgtggacgttcggccaagggaccaaggttga
aatcaaa (SEQ ID NO:71)
2. Fab BU.2
   Hc: 
ctcgagtctgggggaggcctggtcaagcctggggggtccctgagactctcctgtgcagcctctggattcaacttcaatac
ctataccatgaactgggtccgccaggctccagggaaggggctggagtgggtctcatccattagtagtagtagtagttaca
tagacaacgcagactcagtgaagggccgattcaccatctacagagacaacgccaagaagtcactgtatctgcgaatgat
cggcctgagagtcgaggactccggtgtgtattactgtacgcgagtgaacccccaggccaaagggtcggactggttgga
tccccccatcaaccaatactacggtatggacgtctggggccaaagggaccacggtcaccggtctcct
(SEQ ID NO:72)
   Lc: 
gagctcacactcacgcagtctccagccaccctgtctttgtctccaggggatagagccaccctctcctgcagggccagtc
agagtgttagcagcagttacttagcctggtaccagcacaaacctggccagccccccaggctcctcatctatggtgcatcc
agcagggccgccggcatcccagacaggttcagtggcagtgggtctgggacagacttcactctcaccatcagcagact
ggagcctgaagattttgcagtgtattactgtcagcagtacgtcactttcggccctgggaccaaagtggatatcaaa
(SEQ ID NO:73)
3. Fab BU.7
   Hc: 
ctcgagtggggcccaggactggtgaagccttcggagaccctgtccctcacctgcactgtctctggtggctccatcagca
gcataaattactactgggtctggattcgccagcccccagggaaggggctggagtggattgggaatatcaattacagtgg
gaccaccaactacaacccgtccctcaagagtcgagtcaccatatccgtggacacgtccaagaaccagttctccctgaag
ctgagctctgtgaccgccgcagacacggctgtctattactgtgcgagacaaacgtattactatgataggcgtggttattac
cgcccggagcccattgagcactggggccagggaaccctggtcaccgtctcctca (SEQ ID NO:74)
   Lc: 
gagctcgtgatgacacagtctccatccttcctgtctgcatctgtaggagacagagtcaccatcacttgccgggcaagtca
agatgttagaagttttttacattggtatcaacaaagaccagggaaagcccctaagttgttgatctatgctgcatccatggtgt
cgagtgaggtcccgtcaaggttcagtggcagtggatctgagacagatttcactctcaccatcgacggtctgcaacctgaa
gatgttgcaacttacttctgtcaacagacttacgacacgccgctcaccttcggcggcgggaccgcggttgacatcaaa
(SEQ ID NO:75)
4. FabBU.11
   Hc: 
ctcgagtcaggtccaggactgctgaagccctcgcagagcctctcactcacctgtgccatctccggagacagtgtctcta
ggagaagtgttgcttggaactggatcagacagtccccatcgagaggccttgagtggctgggaaggacatactacaggt
ccaagtggttttctgagtatggcgtatctgtgagaggtcgaataaccatcagtccagacacaaccaagaaccagttctcc
ctgcagctgaactccgtgactcccgaggacacggctgtctattactgtcgaaagtcgaaagggaggcagcagttggca
gagtctacgagttcggtatggacgtggggccaagggaccacggtcatcgtctactca (SEQ ID NO:76)
   Lc: 
gagctcgtgatgacacagtctccatcctccctgtctgcatttgtgggagatagagtcaccctcacttgccgggcaagtca
gggcattagaaatgatttaaactggtatcagcagaaaccagggcaaccccctaaactcctgatctatgctgcatccgcttt
acaaagtggcgtcccatcaaggttcagcggcagtgggtttggcacagatttcactctcaccatcagcagcctgcagcct
gaagatattgcaacttattactgtctacaagattacaatttccctcggacgttcggccaagggaccaaggtggaaatcaaa
(SEQ ID NO:77)
5. Fab BU.54
   Hc: 
ctcgagtggggcccaggactggtgaaggcttcacagaccctgtccctcacctgcactgtctctggtggctccatcagca
gtaggaatttctattggagttggatccggcagcccggcgggaagggactggagtggattgggcgtatctatacaagtgg
gagcaccaattacaatccctccctcaagagtcgagtcactatatcattagacacgtccaagagtcagttctccctgaagct
gagctctgtgaccgccgcagacacggccgtgtattattgtgcgagagggacgttttattatgataggagtggtaatggtc
gattagatccgcttgactactggggccagggaaccctggtcaccgtctcctca (SEQ ID NO:78)
   Lc:

gagctcgtgatgacacagtctccatcctccctgtctgcatctgtaggagacagagtcaccatcacttgccaggcgagtca
ggacattagcaactatttaaattggtatcagcagaaaccagggaaagcccctaagctcctgatctacgatgcatccaattt
ggaaacaggggtcccatcaaggttcagtggaagtggatttgggacacattttacgttaaccatcagcagcctgcagcct
gaagattttgcaacatattactgtcaacagcatgataatctcgtcactttcggcggagggaccaaggtggagatcaaa
(SEQ ID NO:79)

**Figure 2** shows the whole amino acid and nucleotide sequence of the heavy chain variable region and of the light chain variable region for each clone, specifying both the framework regions FR1-FR4 and the complementary determining regions CDR1-CDR3.

### EXAMPLE 2: Study on the binding activity of the neutralizing mAbs of the invention Binding evaluation of the Fabs

### a) Enzyme Linked Immunosorbent Assay (ELISA)

The binding efficiency of the Fabs was tested using ELISA assay. 100 ng of the recombinant Spike protein D614G (University of Pavia), B.1.1.7 (40589-V08B6, Sino Biological), P.1 (40589-V08B10, Sino Biological), or B.1.135 (40589-V08B7, Sino Biological) were coated on ELISA plates (CLS3690-100EA, Merck) and incubated overnight at 4°C. 1% bovine serum albumin (BSA; Sigma-Aldrich) solution in PBS was added as a nonspecific reactivity control. The following day, the plate was blocked with a 1% BSA solution in PBS to prevent nonspecific binding to wells, and different dilutions of Fabs were incubated with antigens for 1 h at 37°C. After washing with 0.1% Tween 20 (Sigma-Aldrich) in PBS solution, Fabs were detected with goat anti-human IgG (Fab specific)-peroxidase antibody (A0293; Sigma-Aldrich), with His-tagged proteins with anti-His6-peroxidase antibody (11922416001; Sigma-Aldrich) as a coating control and using the Pierce TMB substrate kit (ThermoFisher Scientific). The OD450 was measured using Multiskan GO (ThermoFisher Scientific).

### RESULTS

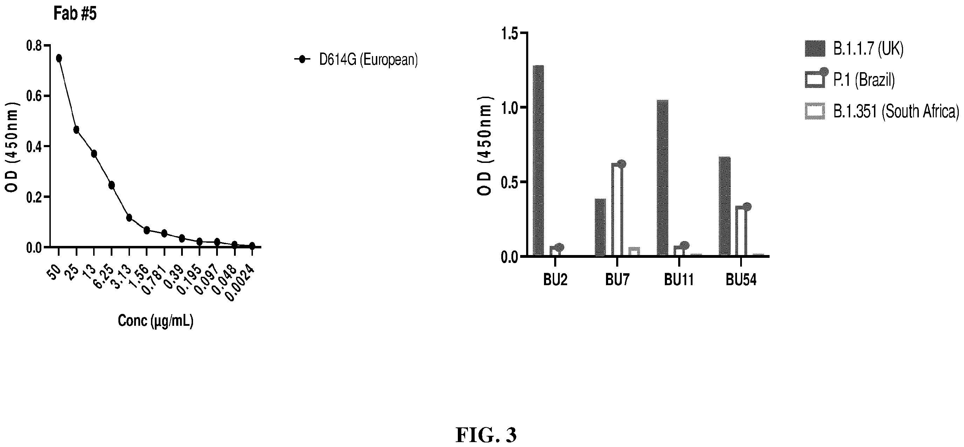

For the Fab #5, a binding curve was tested on D614G spike protein, while Fab BU.2, BU.7, BU.11, BU.54 were tested on the different VOCs at a single concentration **(****Figure 3****).** Data showed that Fab #5 can recognize the recombinant protein even when used at low concentrations, while the BU.7 seems to be the only Fab that weakly binds to B.1.135 spike protein. Moreover, B.1.1.7 spike is well recognized by all BU Fabs, with BU.7 reporting the lowest signal, while P.1 recombinant protein is bound by BU.7 and BU.54, while only a weak signal was observed using BU.2 and BU.11.

### b) Immunofluorescence analysis

### Virus and cells

Vero E6 (Vero C1008, clone E6-CRL-1586; ATCC) cells were cultured in Dulbecco's modified Eagle's medium (DMEM) supplemented with non-essential amino acids (NEAA), penicillin/streptomycin (P/S), Hepes buffer, and 10% (v/v) fetal bovine serum (FBS). Four clinical isolates of SARS-CoV-2 were obtained and propagated in Vero E6 cells: D614G (hCoV-19/Italy/UniSR1/2020; GISAID Accession ID: EPI_ISL_413489), B.1.1.7 (Alpha) (19/Italy/LOM-UniSR7/2021; GSAID Accession ID: EPI_ISL_1924880), C.36_3 (hCoV-19/Italy/LOM-UnINSU/2021, GISAID Accession ID: EPI_ISL_1509923), B.1.351 (Beta) (hCoV-19/Italy/LOM-UniSR6/2021, GISAID Accession ID: EPI_ISL_1599180), P.1 (Gamma) (hCoV-19/Italy/LOM-UniSR8/2021, GISAID Accession ID: EPI_ISL_1925323).

### Virus titration

Virus stocks were titrated using both Plaque Reduction Assay (PRA, PFU/ml) and Endpoint Dilutions Assay (EDA, TCID50/ml). In PRA, confluent monolayers of Vero E6 cells were infected with eight 10-fold dilutions of virus stock. After 1 h of adsorption at 37°C, the cell-free virus was removed. Cells were then incubated for 48 h in DMEM containing 2% FBS and 0.5% agarose. Cells were fixed and stained, and viral plaques were counted. In EDA, Vero E6 cells were seeded into 96 wells plates and infected at 95% of confluency with base 10 dilutions of virus stock. After 1 h of adsorption at 37°C, the cell-free virus was removed, cells were washed with PBS 1×, and complete medium was added to cells. After 48 h, cells were observed to evaluate the presence of a cytopathic effect (CPE). TCID50/ml of viral stocks were then determined by applying the Reed-Muench formula.

### Immunofluorescence assay

Vero E6 cells were seeded into 96 wells plates 24 h before the experiment performed at 95% cell confluency for each well, and then infected with SARS-CoV-2 at 0.01 multiplicity of infection (MOI) for 1 h at 37°C. Then, the cells were washed with PBS 1× to remove cell-free virus particles and virus-containing mixtures and controls were replaced with complete DMEM supplemented with 2% FBS. The plates were incubated at 37°C in the presence of CO2 for 72 h. Then were washed with PBS 1× and fixed with MeOH:Ac (1:1) for 15 minutes, and stored at -20°C. After rinsing the cells with two PBS 1× washes, the selected Fabs (1:200 dilution) were applied to cells as primary antibody. Anti-Spike Antibody (0150-R007, Sino Biological) was added as control. After a PBS 1x wash, Fabs were detected with goat anti-rabbit IgG (H+L) cross-adsorbed secondary antibody, Alexa Fluor 488 (A-11008, Thermo Scientifc). Hoechst 33258 (Sigma-Aldrich) was used for nuclear staining.

### RESULTS

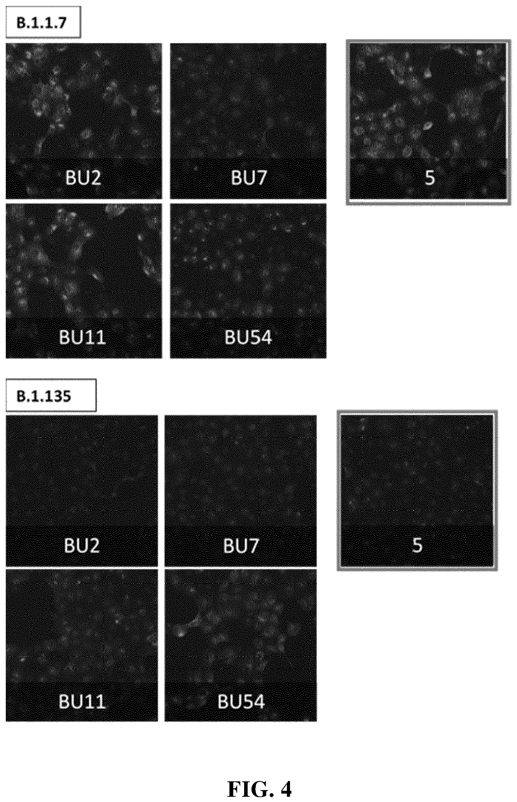

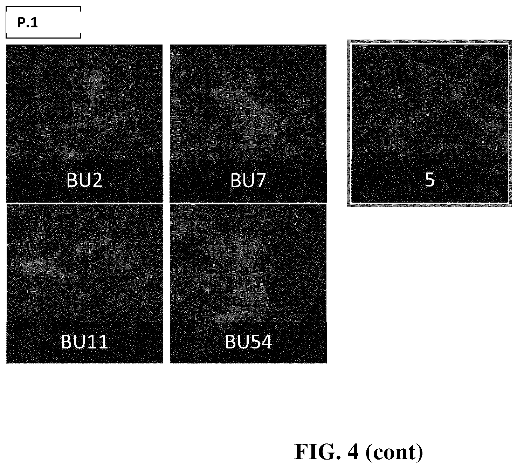

All the selected Fabs detected the infected cells, with slight differences between the tested VOCs **(****Figure 4****).** B.1.1.7 variant is recognized better by Fab #5, BU.2, BU.11 and less well by BU.7 and BU.54. B.1.135 variant is recognized well only by BU.11 and BU.54, while only a slight green signal is observed using the other Fabs. The binding to the P.1 variant is well observed with all the tested Fabs, but the Fab #5 signal had the least intensity.

### EXAMPLE 3:Neutralizing activity evaluation

### Microneutralization experiments

Vero E6 cells were seeded into 96 wells plates 24 h before the experiment performed at 95% cell confluency for each well. Two-fold serial dilutions of the selected clones were incubated with SARS-CoV-2 at 0.01 MOI for 1 h at 37°C. Virus-serum mixtures and positive infection control were applied to Vero E6 monolayers after washing cells with PBS 1×, and virus adsorption was carried out at 37°C for 1 h. Then, the cells were washed with PBS 1× to remove cell-free virus particles and virus-containing mixtures and controls were replaced with complete DMEM supplemented with 2% FBS. The plates were incubated at 37°C in the presence of CO₂ for 72 h. The experiments were performed in triplicate. Neutralization activity was evaluated by comparing cytopathic effect (CPE) presence detected in the presence of virus-serum mixtures to positive infection control.

### RESULTS

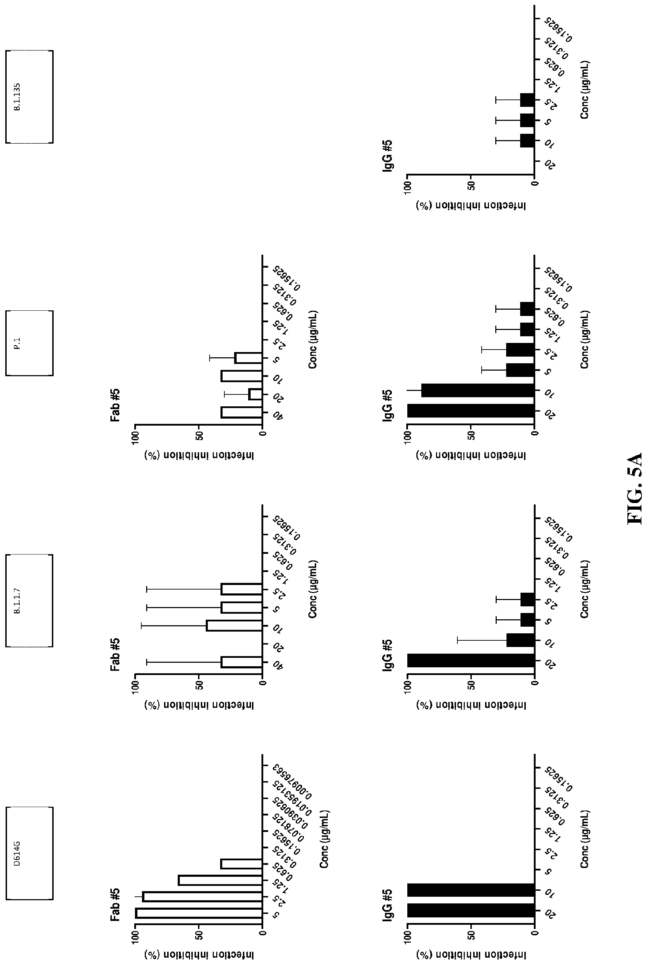

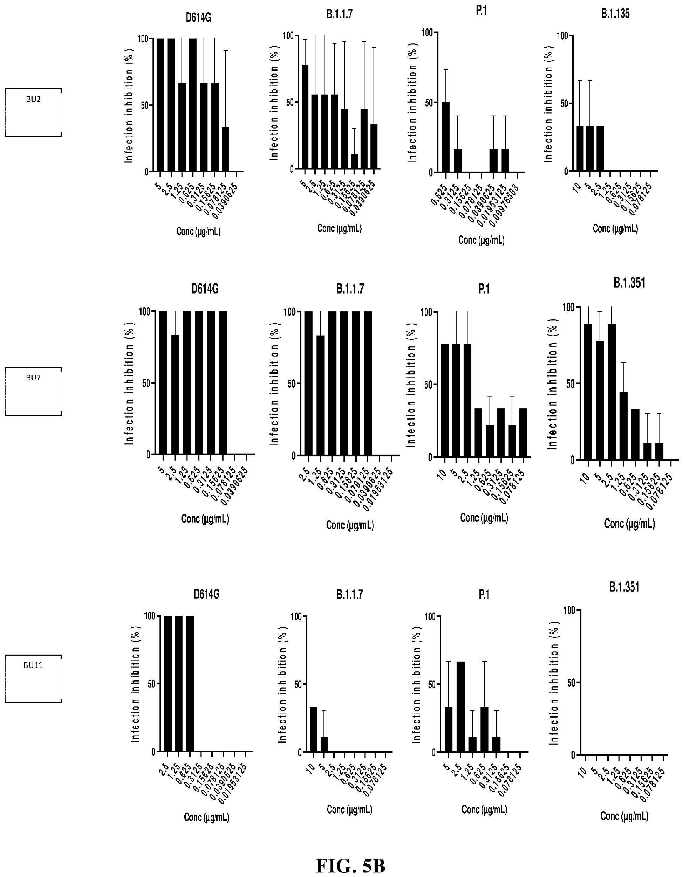

The clone #5 showed an important neutralizing activity against D614G variant both as Fab format and IgG1. However, IgG1showed enhanced activity against the other tested VOCs compared to the Fab fragment, except for B.1.135 variant (Figure 5A). All tested BU clones can inhibit efficiently D614G, but only BU.7 retains its activity against all the tested VOCs. Fab BU.2 can neutralize better B.1.1.7 variant than the other two, while BU.11 showed a significant activity against P.1 (Figure 5B).

The following Tables 2-3 summarize the neutralizing activity expressed as IC₅₀ µg/ml shown by the selected clones Fab #5, IgG #5, BU2, BU7 and BU11 against different VOCs of SARS-Cov-2.

**Table 2**

| | Fab#5 (µg/ml) | IgG#5 (µg/ml) |
|---|---|---|
| D614G | 0.904 | 7.670 |
| B.1.1.7 | 1.422 | 2.177 |
| P.1 | 4.459 | 5.414 |

**Table 3**

| | BU2 (µg/ml) | BU7 (µg/ml) | BU11 (µg/ml) |
|---|---|---|---|
| D614G | 0.002935 | 0.0985 | 0.4075 |
| B.1.1.7 | 5.2 | 0.67425 | 5.3525 |
| P.1 | 0.31205 | 0.4426 | 0.3182 |
| B.1.351 | 35.57 | 0.955 | NA |

### REFERENCES

1. Lanying Du et al. (2021) Neutralizing antibodies for the prevention and treatment of COVID-19. Cellular and Molecular Immunology, 18, pp. 2293-2306. doi:10.1038/s41423-021-00752-2
2. An EUA for Bamlanivimab-A Monoclonal Antibody for COVID-19. JAMA. 2021;325(9):880-881. doi:10.1001/jama.2020.24415
3. An EUA for bamlanivimab and etesevimab for COVID-19. Med Lett Drugs Ther. 2021 Apr 5;63(1621):49-50. pmid:33830966
4. An EUA for casirivimab and imdevimab for COVID-19. Med Lett Drugs Ther. 2020 Dec 28;62(1614):201-2. pmid:33451174
5. Dong J, Zost SJ, Greaney AJ, Starr TN, Dingens AS, Chen EC, et al. Genetic and structural basis for recognition of SARS-CoV-2 spike protein by a two-antibody cocktail. bioRxiv; doi:10.1101/2021.01.27.428529
6. Cathcart AL, Havenar-Daughton C, Lemp FA, Ma D, Schmid MA, Agostini ML, et al. The dual function monoclonal antibodies VIR-7831 and VIR-7832 demonstrate potent in vitro and in vivo activity against SARS-CoV-2. bioRxiv; doi: 10.1101/2021.03.09.434607
7. Kim C, Ryu D-K, Lee J, Kim Y-I, Seo J-M, Kim Y-G, et al. A therapeutic neutralizing antibody targeting receptor binding domain of SARS-CoV-2 spike protein. Nat Commun. 2021 Jan 12;12(1):288. doi:10.1038/s41467-020-20602-5
8. Alsoussi WB, Turner JS, Case JB, Zhao H, Schmitz AJ, Zhou JQ, et al. A Potently Neutralizing Antibody Protects Mice against SARS-CoV-2 Infection. J Immunol. 2020 Aug 15;205(4):915-22. doi:10.4049/jimmunol.2000583
9. Wang C, Li W, Drabek D, Okba NMA, van Haperen R, Osterhaus ADME, et al. A human monoclonal antibody blocking SARS-CoV-2 infection. Nat Commun. 2020 May 4;11(1):2251. doi:10.1038/s41467-020-16256-y
10. Tzou PL, Tao K, Nouhin J, Rhee S-Y, Hu BD, Pai S, et al. Coronavirus Antiviral Research Database (CoV-RDB): An Online Database Designed to Facilitate Comparisons between Candidate Anti-Coronavirus Compounds. Viruses. 2020 Sep;12(9):1006. doi:10.3390/v12091006
11. Kumar S. et al. (2021) Current status of therapeutic monoclonal antibodies against SARS-CoV-2. PLoS Pathog 17(9): e1009885. doi:10.1371/journal.ppat.1009885
12. Frampton D, Rampling T, Cross A, Bailey H, Heaney J, Byott M, et al. Genomic characteristics and clinical effect of the emergent SARS-CoV-2 B.1.1.7 lineage in London, UK: a whole-genome sequencing and hospital-based cohort study. Lancet Infect Dis. 2021 Sep;21(9):1246-1256. doi:10.1016/S1473-3099(21)00170-5.
13. Tegally H, Wilkinson E, Giovanetti M, Iranzadeh A, Fonseca V, Giandhari J, et al. Emergence and rapid spread of a new severe acute respiratory syndrome-related coronavirus 2 (SARS-CoV-2) lineage with multiple spike mutations in South Africa. medRxiv; doi: 10.1101/2020.12.21.20248640
14. Voloch CM, da Silva FR, de Almeida LGP, Cardoso CC, Brustolini OJ, Gerber AL, et al. Genomic characterization of a novel SARS-CoV-2 lineage from Rio de Janeiro, Brazil. J Virol. 2021 Mar 1. doi:10.1128/JVI.00119-21
15. Singh J, Rahman SA, Ehtesham NZ, Hira S, Hasnain SE. SARS-CoV-2 variants of concern are emerging in India. Nat Med. 2021 Jul;27(7):1131-3. doi:10.1038/s41591-021-01397-4
16. West AP, Wertheim JO, Wang JC, Vasylyeva TI, Havens JL, Chowdhury MA, et al. Detection and characterization of the SARS-CoV-2 lineage B.1.526 in New York. bioRxiv. doi:10.1101/2021.02.14.431043
17. Wink PL, Volpato FCZ, Monteiro FL, Willig JB, Zavascki AP, Barth AL, et al. First identification of SARS-CoV-2 Lambda (C.37) variant in Southern Brazil. medRxiv. doi:10.1101/2021.06.21.21259241
18. Younes A, Bartlett NL, Leonard JP, et al. Brentuximab vedotin (SGN-35) for relapsed CD30-positive lymphomas. N Engl J Med 2010; 363: 1812-21. Doi:10.1056/NEJMoa1002965
19. Petersdorf SH, Kopecky KJ, Slovak M, Willman C, Nevill T, Brandwein J, Larson RA, Erba HP, Stiff PJ, Stuart RK, Walter RB, Tallman MS, Stenke L, Appelbaum FR. A phase 3 study of gemtuzumab ozogamicin during induction and postconsolidation therapy in younger patients with acute myeloid leukemia. Blood. 2013 Jun 13;121(24):4854-60. doi:10.1182/blood-2013-01-466706.
20. Olsen E, Duvic M, Frankel A, et al. Pivotal phase III trial of two dose levels of denileukin diftitox for the treatment of cutaneous T-cell lymphoma. J Clin Oncol 2001; 19: 376-88. doi:10.1200/JCO.2001.19.2.376
21. Kreitman RJ, Tallman MS, Robak T, et al. Ph ase I trial of anti-CD22 recombinant immunotoxin moxetumomab pasudotox (CAT-8015 or HA22) in patients with hairy cell leukemia. J Clin Oncol 2012; 30: 1822-28. Doi:10.1200/JC0.2011.38.1756
22. Pruszynski M, Koumarianou E, Vaidyanathan G, Revets H, Devoogdt N, Lahoutte T, et al. Improved tumor targeting of anti-HER2 nanobody through N-Succinimidyl 4-Guanidinomethyl-3-iodobenzoate radiolabeling. J Nuclear Med 2014;55:650-6. doi:10.2967/jnumed.113.127100

## Claims

1. An antibody or antibody fragment binding a conserved region of the spike protein of a Sarbecovirus, **characterized by** an antigen binding site comprising:
a) a heavy chain variable sequence comprising:
i) a CDR1 comprising an amino acid sequence selected from the group consisting of SEQ ID NO:1, SEQ ID NO:2, SEQ ID NO:3 and SEQ ID NO:4;
ii) a CDR2 comprising an amino acid sequence selected from the group consisting of SEQ ID NO:5, SEQ ID NO:6, SEQ ID NO:7 and SEQ ID NO:8;
iii) a CDR3 comprising an amino acid sequence selected from the group consisting of SEQ ID NO:9, SEQ ID NO:10, SEQ ID NO:11 and SEQ ID NO:12; and/or
b) a light chain variable sequence comprising:
i) a CDR1 comprising an amino acid sequence selected from the group consisting of SEQ ID NO:13, SEQ ID NO:14, SEQ ID NO:15 and SEQ ID NO:16;
ii) a CDR2 comprising an amino acid sequence selected from the group consisting of GAS, AAS and DAS;
iii) a CDR3 comprising an amino acid sequence selected from the group consisting of SEQ ID NO:20, SEQ ID NO:21, SEQ ID NO:22, SEQ ID NO:23 and SEQ ID NO:24.

2. An antibody or antibody fragment according to claim 1, wherein the heavy chain variable sequence further comprises:
1) a framework region (FR1) comprising an amino acid sequence selected from the group consisting of SEQ ID NO:25, SEQ ID NO:26, SEQ ID NO:27 and SEQ ID NO:28;
2) a framework region (FR2) comprising an amino acid sequence selected from the group consisting of SEQ ID NO:29, SEQ ID NO:30, SEQ ID NO:31 and SEQ ID NO:32;
3) a framework region (FR3) comprising an amino acid sequence selected from the group consisting of SEQ ID NO:33, SEQ ID NO:34, SEQ ID NO:35 and SEQ ID NO:36;
4) a framework region (FR4) comprising an amino acid sequence selected from the group consisting of SEQ ID NO:37, SEQ ID NO:38, SEQ ID NO:39 and SEQ ID NO:40;
and/or
wherein the light chain variable sequence further comprises:
5) a framework region (FR1) comprising an amino acid sequence selected from the group consisting of SEQ ID NO:41, SEQ ID NO:42, SEQ ID NO:43; SEQ ID NO:44 and SEQ ID NO:45;
6) a framework region (FR2) comprising an amino acid sequence selected from the group consisting of SEQ ID NO:46, SEQ ID NO:47, SEQ ID NO:48, SEQ ID NO:49 and SEQ ID NO:50;
7) a framework region (FR3) comprising an amino acid sequence selected from the group consisting of SEQ ID NO:51, SEQ ID NO:52, SEQ ID NO:53, SEQ ID NO:54 and SEQ ID NO:55;
8) a framework region (FR4) comprising an amino acid sequence selected from the group consisting of SEQ ID NO:56, SEQ ID NO:57, SEQ ID NO:58 and SEQ ID NO:59.

3. An antibody or antibody fragment according to anyone of claim 1-2 wherein the heavy chain variable sequence and/or light chain sequence comprises the following combination of CDRs:
a) CDR1 comprising or consisting of the amino acid sequence SEQ ID NO:1; CDR2 comprising or consisting of the amino acid sequence SEQ ID NO:5; CDR3 comprising or consisting of the amino acid sequence SEQ ID NO:9 for the heavy chain variable sequence; CDR1 comprising or consisting of the amino acid sequence SEQ ID NO:13; CDR2 comprising or consisting of the amino acid sequence GAS; CDR3 comprising or consisting of the amino acid sequence SEQ ID NO:20 for the light chain variable sequence;
b) CDR1 comprising or consisting of the amino acid sequence SEQ ID NO:1; CDR2 comprising or consisting of the amino acid sequence SEQ ID NO:5; CDR3 comprising or consisting of the amino acid sequence SEQ ID NO:9 for the heavy chain variable sequence; CDR1 comprising or consisting of the amino acid sequence SEQ ID NO:13; a CDR2 comprising or consisting of the amino acid sequence GAS; a CDR3 comprising or consisting of the amino acid sequence SEQ ID NO:21 for the light chain variable sequence;
c) CDR1 comprising or consisting of the amino acid sequence SEQ ID NO:2; CDR2 comprising or consisting of the amino acid sequence SEQ ID NO:6; a CDR3 comprising or consisting of the amino acid sequence SEQ ID NO:10 for the heavy chain variable sequence; CDR1 comprising the amino acid sequence SEQ ID NO:14; a CDR2 comprising the amino acid sequence AAS; a CDR3 comprising the amino acid sequence SEQ ID NO:22 for the heavy chain variable sequence;
d) CDR1 comprising or consisting of the amino acid sequence SEQ ID NO:3; a CDR2 comprising or consisting of the amino acid sequence SEQ ID NO:7; a CDR3 comprising or consisting of the amino acid sequence SEQ ID NO:11 for the heavy chain variable sequence; CDR1 comprising or consisting of the amino acid sequence SEQ ID NO:15; a CDR2 comprising or consisting of the amino acid sequence AAS; a CDR3 comprising or consisting of the amino acid sequence SEQ ID NO:23 for the light chain variable sequence;
e) CDR1 comprising or consisting of the amino acid sequence SEQ ID NO:4; CDR2 comprising or consisting of the amino acid sequence SEQ ID NO:8; CDR3 comprising or consisting of the amino acid sequence SEQ ID NO:12 for the heavy chain variable sequence; CDR1 comprising or consisting of the amino acid sequence SEQ ID NO:16; CDR2 comprising or consisting of the amino acid sequence DAS; CDR3 comprising or consisting of the amino acid sequence SEQ ID NO:24.

4. An antibody or antibody fragment according to claim 3, wherein said heavy chain variable sequence and/or said light chain variable sequence further comprises the following combination of FRs:
a) FR1 comprising or consisting of SEQ ID NO:25; FR2 comprising or consisting of SEQ ID NO:29; FR3 comprising or consisting of SEQ ID NO:33 and FR4 comprising or consisting of SEQ ID NO:37 for the heavy chain variable sequence; FR1 comprising or consisting of SEQ ID NO:41; FR2 comprising or consisting of SEQ ID NO:46; FR3 comprising or consisting of SEQ ID NO:51 and a framework region FR4 comprising or consisting of SEQ ID NO:56 for the light chain variable sequence;
b) FR1 comprising or consisting of SEQ ID NO:25; FR2 comprising or consisting of SEQ ID NO:29; FR3 comprising or consisting of SEQ ID NO:33 and FR4 comprising or consisting of SEQ ID NO:38 for the heavy chain variable sequence; FR1 comprising or consisting of SEQ ID NO:42; FR2 comprising or consisting of SEQ ID NO:47; FR3 comprising or consisting of SEQ ID NO:52, and FR4 comprising or consisting of SEQ ID NO:57 for the light chain variable sequence;
c) FR1 comprising or consisting of SEQ ID NO:26; FR2 comprising or consisting of SEQ ID NO:30; FR3 comprising or consisting of SEQ ID NO:34 and FR4 comprising or consisting of SEQ ID NO:39 for the heavy chain variable sequence; FR1 comprising or consisting of SEQ ID NO:43; FR2 comprising or consisting of SEQ ID NO:48; FR3 comprising or consisting of SEQ ID NO:53, and FR4 comprising or consisting of SEQ ID NO:58 for the light chain variable sequence;
d) FR1 comprising or consisting of SEQ ID NO:27; FR2 comprising or consisting of SEQ ID NO:31; FR3 comprising or consisting of SEQ ID NO:35 and FR4 comprising or consisting of SEQ ID NO:40 for the heavy chain variable sequence; FR1 comprising or consisting of SEQ ID NO:44; FR2 comprising or consisting of SEQ ID NO:49; FR3 comprising or consisting of SEQ ID NO:54, and FR4 comprising or consisting of SEQ ID NO:56 for the light chain variable sequence;
e) FR1 comprising or consisting of SEQ ID NO:28; FR2 comprising or consisting of SEQ ID NO:32; FR3 comprising or consisting of SEQ ID NO:36; FR4 comprising or consisting of SEQ ID NO:39 for the heavy chain variable sequence; FR1 comprising or consisting of SEQ ID NO:45; FR2 comprising or consisting of SEQ ID NO:50; FR3 comprising or consisting of SEQ ID NO:55 and a FR4 comprising or consisting of SEQ ID NO:59 for the light chain variable sequence.

5. An antibody or antibody fragment according to anyone of claims 1-4, which comprises or consists of:
a) an heavy chain variable sequence comprising or consisting of the amino acid sequence SEQ ID NO:60 and/or a light chain variable sequence comprising or consisting of the amino acid sequence SEQ ID NO: 61;
or
b) an heavy chain variable sequence comprising or consisting of the amino acid sequence SEQ ID NO: 62 and/or a light chain variable sequence comprising or consisting of the amino acid sequence SEQ ID NO: 63;
c) an heavy chain variable sequence comprising or consisting of the amino acid sequence SEQ ID NO:64 and/or a light chain variable sequence comprising or consisting of the amino acid sequence SEQ ID NO:65;
d) an heavy chain variable sequence comprising or consisting of the amino acid sequence SEQ ID NO:66 and/or a light chain variable sequence comprising or consisting of the amino acid sequence SEQ ID NO:67;
e) an heavy chain variable sequence comprising or consisting of the amino acid sequence SEQ ID NO:68 and/or a light chain variable sequence comprising or consisting of the amino acid sequence SEQ ID NO:69.

6. An antibody or antibody fragment according to anyone of claim 1-4 which is a monoclonal antibody, preferably a human monoclonal antibody.

7. An antibody or antibody fragment according to anyone of claim 1-5 which is an IgG selected from the group consisting of IgG1, IgG2, IgG3, IgG4; a Fab fragment; a single chain antibody (scFv); a bispecific antibody; optionally conjugated with a drug or a label.

8. A nucleotide sequence encoding for the amino acid sequence of the heavy chain variable region and/or the light chain variable region of the antibody or antibody fragment according to anyone of claims 1-7.

9. An expression vector comprising at least one nucleotide sequence according to claim 8.

10. An host cell comprising the expression vector according to claim 9.

11. An hybridoma comprising at least one of the nucleotide sequence according to claim 8 for the production of the antibody or antibody fragment according to anyone of claims 1-7.

12. A formulation for molecular/vector-based passive immunoprophylaxis comprising at least one of the nucleotide sequence or expression vector according to anyone of claims 8-9 together with one or more pharmaceutically acceptable excipients and/or adjuvants.

13. An antibody or antibody fragment according to anyone of the claims 1-7, for use in medical field for the treatment and/or prophylaxis of Sarbecoviruses-mediated diseases in a subject, in particular of the Severe Acute Respiratory Syndrome mediated by SARS-CoV-2 and variants of concern thereof.

14. An antibody or antibody fragment for use according to claim 13, wherein said variants of concern are selected among the group comprising Alpha (B.1.1.7), Beta (B.1.351) Gamma (P.1), Delta (B.1.617.2) and D614 G (Università di Pavia) variants of SARS-CoV-2.

15. An antibody or antibody fragment for use according to anyone of the claims 13-14, wherein the subject to be treated is an immunocompromised patient, a patient with an history of cardiovascular and/or respiratory diseases, an elderly patient or a vaccine-hesitant subject.

16. An antibody or antibody fragment for use according to anyone of the claims 13-15, wherein a first antibody or antibody fragment is used alone or in combination with a second antibody or antibody fragment directed against a different conserved region of the spike protein.

17. A pharmaceutical composition comprising at least one of the antibody or antibody fragment of the invention as the active ingredient, together with one or more pharmaceutically acceptable excipients and/or adjuvants.

18. A pharmaceutical composition according to claim 17, which is suitable for intravenous, intramuscular or subcutaneous administration.

19. A composition or kit of parts comprising a first antibody or antibody fragment that binds a targeted conserved region of the spike protein of SARS-CoV-2 and a second antibody or antibody fragment that binds a different targeted conserved region of the spike protein of SARS-CoV-2 with respect to the first antibody, for the simultaneous, separate or sequential administration in a subject affected by Sarbecoviruses-mediated diseases, in particular by the Severe Acute Respiratory Syndrome mediated by SARS-CoV-2.

20. Use of an antibody or antibody fragment according to anyone of the claims 1-7 to detect the presence of a Sarbecovirus, preferably of SARS-CoV-2 in a biological sample.
